# EUROPÄISCHE PATENTSCHRIFT

(11) **EP 3 079 652 B1**
(45) Veröffentlichungstag und Bekanntmachung des Hinweises auf die Patenterteilung: **27.12.2017**
(21) Anmeldenummer: 14786441.7
(22) Anmeldetag: 05.09.2014
(51) Int. Cl.: A61K 8/44, A61Q 5/04, A45D 7/06

(54) **HAARGLÄTTUNG MIT CARBOCYSTEIN**
HAIR STRAIGHTENING WITH CARBOCISTEINE
DÉFRISAGE AVEC CARBOCYSTÉIN

(30) Priorität: 13.12.2013 DE 102013225916
(43) Veröffentlichungstag der Anmeldung: 19.10.2016
(73) Patentinhaber: Henkel AG & Co. KGaA, 40589 Düsseldorf (DE)
(72) Erfinder: RAUTENBERG-GROTH, Birgit, 25479 Ellerau (DE); SCHWARTZ, Stephan, 22880 Wedel (DE); KRAUSE, Katharina, 22041 Hamburg (DE)
(86) Internationale Anmeldenummer: PCT/DE2014/200445
(87) Internationale Veröffentlichungsnummer: WO 2015/085997

(56) Entgegenhaltungen:
- WO-A1-2007/128983
- DE-A1-102011 089 573
- DE-A1-102012 222 286
- US-A- 4 172 887

## Beschreibung

Die Erfindung betrifft das technische Gebiet der Umformung keratinhaltiger Fasern, insbesondere menschlicher Haare. Gegenstand der Erfindung ist ein verbessertes Verfahren zur Umformung keratinhaltiger Fasern, insbesondere menschlicher Haare. Als keratinhaltige Fasern können prinzipiell alle tierischen Haare, z.B. Wolle, Roßhaar, Angorahaar, Pelze, Federn und daraus gefertigte Produkte oder Textilien eingesetzt werden. Bevorzugt wird die Erfindung jedoch im Rahmen einer Haarumformung, insbesondere der Glättung krauser menschlicher Haare sowie daraus gefertigter Perücken, eingesetzt.

Eine dauerhafte Verformung keratinhaltiger Fasern wird üblicherweise derart durchgeführt, dass man die Faser mechanisch verformt und die Verformung durch geeignete Hilfsmittel festlegt. Vor und/oder nach dieser Verformung behandelt man die Faser mit einer keratinreduzierenden Zubereitung. Nach einem Spülvorgang wird die Faser dann in dem so genannten Fixierschritt mit einer Oxidationsmittelzubereitung behandelt, gespült und nach oder während des Fixierschritts von den Verformungshilfsmitteln (z.B. Wicklern, Papilloten) befreit. Wenn als keratinreduzierende Komponente ein Merkaptan, z.B. Ammoniumthioglykolat, verwendet wird, spaltet dieses einen Teil der Disulfid-Brücken des Keratin-Moleküls zu -SH-Gruppen, so dass es zu einer Erweichung der Keratinfaser kommt. Bei der späteren oxidativen Fixierung werden erneut Disulfid-Brücken im Haarkeratin geknüpft, so dass das Keratingefüge in der vorgegebenen Verformung fixiert wird. Alternativ ist es bekannt, zur Haarverformung anstelle der Merkaptane Sulfit zu verwenden. Durch Hydrogensulfit-Lösungen und/oder Sulfit-Lösungen und/oder Disulfit-Lösungen werden Disulfid-Brücken des Keratins in einer Sulfitolyse nach der Gleichung

R-S-S-R + HSO_{3⁽⁻⁾} → R-SH +R-S-SO_{3⁽⁻⁾}

gespalten und auf diese Weise eine Erweichung der Keratinfaser erreicht. Hydrogensulfit-, sulfit- bzw. disulfithaltige Reduktionsmittel weisen nicht den starken Eigengeruch der merkaptanhaltigen Mittel auf. Die Spaltung kann wie zuvor geschildert in einem Fixierschritt mit Hilfe eines Oxidationsmittels unter Bildung von neuen Disulfid-Brücken wieder rückgängig gemacht werden.

Die permanente Glättung keratinhaltiger Fasern wird analog durch den Einsatz von keratinreduzierenden und -oxidierenden Zusammensetzungen erzielt. In einem entsprechenden Verfahren wird das krause Haar entweder auf Wickler mit einem großen Durchmesser von üblicherweise mehr als 15 mm gewickelt oder das Haar unter Einwirkung der keratinreduzierenden Zusammensetzung glattgekämmt. Anstelle des Wicklers ist es auch möglich, die Faser auf ein Glättungsboard glattzulegen. Glättungsboarde sind üblicherweise rechteckige Tafeln z.B. aus Kunststoff. Vorzugsweise ist die Faser dabei mit der keratinreduzierenden Zubereitung benetzt.

Eine weitere Möglichkeit der Haarglättung ist das Glätten mit einem heißen Eisen. Allerdings verändert sich die Struktur der keratinhaltigen Faser bei der Wärmebehandlung des Haars während des Glättens. Dieser Änderung der Faserstruktur sollte durch geeignete Maßnahmen entgegengewirkt werden.

Die Glättung mittels Glätteisen kann durch vorherige Anwendung alkalischer Produkte unterstützt werden. Solche alkalischen Umformumgsmittel führen im Gegensatz zur Umformung mittels keratinreduzierender und -oxidierender Zusammensetzungen nicht zu einer Umformung der Disulfidbrücken, sondern zu einer Zerstörung der Disulfidbrücken unter Bildung von Monosulfidbrücken. Je nach Konzentration und Anwendungsdauer der alkalischen Umformungsmittel werden auch Proteinketten hydrolytisch gespalten. Der pH-Wert der alkalischen Umformungsmittel liegt üblicherweise im Bereich von 11-14, vorzugsweise von 12-13.

Im Allgemeinen haben die bekannten Umformungsverfahren, besonders bei der Glättung, den Nachteil, daß sich die keratinhaltige Faser elektrostatisch auflädt. Darüber hinaus führt die Behandlung mit Umformungsmitteln zu einer gesteigerten Hydrophilität der Haare, was die Frisierbarkeit erschwert und den Griff, die Kämmbarkeit sowie den Glanz verschlechtert.

Aufgabe der Erfindung ist es daher, ein Umformungsverfahren für keratinhaltige Fasern, insbesondere für menschliches Haar, bereitzustellen, das ein sehr gutes und dauerhaftes Umformungsergebnis liefert und gleichzeitig die elektrostatische Aufladung sowie Hydrophilierung des Haares minimiert, die Faser pflegt sowie die Struktur der Faser schont.

Überraschenderweise wurde gefunden, daß eine Behandlung der Fasern mit speziellen Umformungsmitteln vor dem Umformungsvorgang die negativen Folgen der Umformung deutlich minimiert und insbesondere die elektrostatische Aufladung sowie Hydrophilierung des Haares minimiert.

Gegenstand der Offenbarung ist ein Verfahren zur Umformung, insbesondere zur Glättung, keratinhaltiger Fasern, insbesondere menschlicher Haare, in welchem
(i) ein Umformungsmittel, enthaltend Carbocystein und/oder seine Salze auf die keratinischen Fasern aufgetragen und dort belassen wird
(ii) die Fasern nach einer Einwirkzeit
   - nicht gespült werden,
   - gegebenenfalls getrocknet werden,
(iii) die Fasern unter der Einwirkung von Wärme mechanisch verformt werden.

Im erfindungsgemäßen Verfahren wird zunächst ein Umformungsmittel, enthaltend Carbocystein und/oder seine Salze auf die keratinischen Fasern aufgetragen und dort belassen. Dieser Schritt (i) des erfindungsgemäßen Verfahrens kann unmittelbar, d.h. wenige Sekunden bis Minuten vor der eigentlichen Umformungsbehandlung erfolgen, es kann aber auch länger gewartet werden, sofern dies im Einzelfall erwünscht sein sollte. Die bevorzugte Einwirkzeit in Schritt (ii) beträgt 30 Sekunden bis 15 Minuten. Vorzugsweise liegt die Einwirkzeit in Schritt (ii) des erfindungsgemäßen Verfahrens bei 30 Sekunden bis 10 Minuten, weiter bevorzugt bei 1 bis 5 Minuten und insbesondere bei 90 bis 240 Sekunden.

Das in Schritt (i) aufgetragene Umformungsmittel wird nach der Einwirkzeit nicht ausgespült, sondern verbleibt auf der Faser. Die Faser kann in Schritt (ii) optional getrocknet werden, was bei längeren Einwirkzeiten des Umformungsmittels durch Lufttrocknung erfolgen kann. Bei kürzeren Applikationszeiten kann das Haar beispielsweise mit einem Handtuch frottiert oder vorzugsweise gefönt werden. Nach Abschluß eines Frottierschritts bleibt eine fühlbare Restfeuchtigkeit im Haar zurück.

Anschließend werden die Fasern in Schritt (iii) mechanisch verformt, was bei Raumtemperatur oder auch unter Anwendung von Wärme geschehen kann.

Die im erfindungsgemäßen Verfahren eingesetzten Umformungsmittel enthalten Carbocystein und/oder seine Salze. Carbocystein, auch als *S*-Carboxymethyl-L-cystein oder (*R*)-2-Amino-4-thia-adipinsäure bzw L-2-Amino-3-(carboxymethylthio) propionsäure bezeichnet, wird durch die Formel 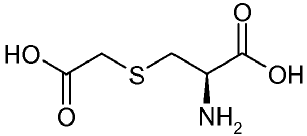 beschrieben, geeignete Salze sind vor allem die Mono- und Di-Alkalisalze, insbesondere Mononatrium-Carbocysteinat, Dinatriumcarbocysteinat, Monokalium-Carbocysteinat und Dikaliumcarbocysteinat.

Erfindungsgemäß bevorzugte Verfahren sind dadurch gekennzeichnet, daß im Schritt (i) ein Umformungsmittel aufgetragen wird, welches bezogen auf sein Gewicht 0,01 bis 10 Gew.-%, vorzugsweise 0,1 bis 7,5 Gew.-% weiter bevorzugt 0,25 bis 5 Gew.-%, noch weiter bevorzugt 0,5 bis 2,5 Gew.-% und insbesondere 0,55 bis 2 Gew.-% Carbocystein enthält.

Die im erfindungsgemäßen Verfahren eingesetzten Umformungsmittel sind wasserbasiert. Sie enthalten Wasser in Mengen oberhalb von 40 Gew.-%, jeweils bezogen auf das Gesamtgewichts des Umformungsmittels, wobei erfindungsgemäß bevorzugte Verfahren dadurch gekennzeichnet sind, daß im Schritt (i) ein Umformungsmittel aufgetragen wird, welches bezogen auf sein Gewicht 40 bis 92,5 Gew.-% vorzugsweise 50 bis 90 Gew.-%, noch weiter bevorzugt 60 bis 87,5 Gew.-% und insbesondere 70 bis 85 Gew.-% Wasser enthält. Die erfindungsgemässen Umformungsmittel enthalten weiter zwingend 0,15 - 5 Gew.-% einer quartären Ammoniumverbindung, wie in Anspruch 1 aufgelistet. Die im erfindungsgemäßen Verfahren eingesetzten Umformungsmittel können weitere Inhaltsstoffe enthalten, wobei sich der Einsatz von Fettstoff(en) und kationischen Verbindungen als besonders geeignet erweisen hat.

Mit besonderem Vorzug enthalten die im erfindungsgemäßen Verfahren eingesetzten Umformungsmittel Fettstoffe (Fat) als weiteren Wirkstoff. Unter Fettstoffen (Fat) sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettsäuren (Fatac) können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können. Als Fettalkohole (Fatal) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse (Fatwax) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an Öl- und Fettkomponenten in den im erfindungsgemäßen Verfahren eingesetzten Umformungsmitteln beträgt üblicherweise 0,5 - 75 Gew.-%, bezogen auf das gesamte Mittel. Mengen von 0,5 - 35 Gew.-% sind erfindungsgemäß bevorzugt.

Bevorzugte Fettstoffe sind lineare und verzweigte, gesättigte und ungesättigte Fettalkohole oder natürliche Fettalkoholgemische mit 8 bis 22 Kohlenstoffatomen in der Alkylkette wie beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole sowie Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden und somit ein Gemisch von unterschiedlichen Fettalkoholen darstellen. Die Fettalkohole werden üblicherweise in Mengen von 0,01 bis 15 Gew.-%, bevorzugt von 0,1 bis 10 Gew.-% und besonders bevorzugt von 0,3 bis 6 Gew.-%, bezogen auf die gesamte Zubereitung, eingesetzt.

Ebenfalls als Fettstoffe eingesetzt werden können Monoester der Fettsäuren mit Alkoholen mit 6 bis 24 C-Atomen sowie Triglyceride natürlichen Ursprungs. Wie weiter oben schon eräwhnt sind erfindungsgemäße Verfahren dadurch gekennzeichnet, daß im Schritt (i) ein Umformungsmittel aufgetragen wird, welches bezogen auf sein Gewicht 0,15 bis 5 Gew.-%, vorzugsweise 0,2 bis 4 Gew.-%, noch weiter bevorzugt 0,25 bis 3 Gew.-% und insbesondere 0,3 bis 1 Gew.-% quartäre Ammoniumverbindung(en) aus der Gruppe
i) der Tetraalkylammoniumhalogenide und/oder
ii) der Esterquats und/oder
iii) der quarternären Imidazoline der Formel (Tkat2) 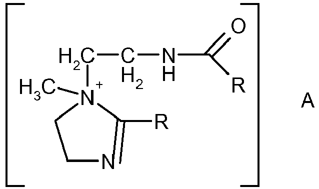 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
v) Poly(methacryloyloxyethyltrimethylammoniumverbindungen) und/oder;
vi) quaternisierten Cellulose-Derivaten, insbesondere Polyquaternium 10, Polyquaternium-24, Polyquaternium-27, Polyquaternium-67, Polyquaternium-72, und/oder
vii) kationische Alkylpolyglycoside und/oder
viii) kationisiertem Honig und/oder
ix) kationischen Guar-Derivaten und/oder
x) polymeren Dimethyldiallylammoniumsalzen und deren Copolymeren mit Estern und Amiden von Acrylsäure und Methacrylsäure, insbesondere Polyquaternium-7 und/oder
xi) Copolymeren des Vinylpyrrolidons mit quaternierten Derivaten des Dialkylaminoalkylacrylats und -methacrylats, insbesondere Polyquaternium-11 und/oder
xii) Vinylpyrrolidon-Vinylimidazoliummethochlorid-Copolymeren, insbesondere Polyquaternium-16 und/oder
xiii) quaterniertem Polyvinylalkohol und/oder
xiv) Polyquaternium-74,
xv) kationischen Alkyloligoglucosiden und/oder
xvi) Polyquaternium-71
sowie deren Mischungen enthält.

Esterquats gemäß der Formel (Tkat1-2) sind die erste Gruppe der quaternären Ammoniumverbindungen.

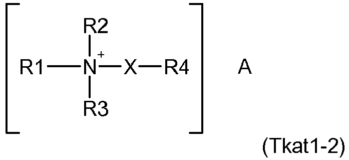

Hierin sind die Reste R1, R2 und R3 jeweils unabhängig voneinander und können gleich oder verschieden sein. Die Reste R1, R2 und R3 bedeuten:
- ein verzweigter oder unverzweigter Alkylrest mit 1 bis 4 Kohlenstoffatomen, welcher mindestens eine Hydroxylgruppe enthalten kann, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- X - R4), mit der Maßgabe, dass höchstens 2 der Reste R1, R2 oder R3 für diesen Rest stehen können:
   Der Rest -(X - R4) ist mindestens 1 bis 3 mal enthalten.

Hierin steht X für:
1) -(CH2)n- mit n = 1 bis 20, vorzugsweise n = 1 bis 10 und besonders bevorzugt n = 1 - 5, oder
2) -(CH2-CH R5-O)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl,
3) eine Hydroxyalkylgruppe mit ein bis vier Kohlenstoffatomen, welche verzweigt oder unverzweigt sein kann, und welche mindestens eine und höchstens 3 Hydroxygruppen enthält. Beispiele für -X- sind: -CHOH, -CHCH₂OH, -CH₂CHOH, -COHCHOH, -CHOHCOH, -CHCHOHCH₃, -CH₂COHCH₃, -CH₂CHOHCH₂-, -C(CH₂OH)₂, -CH₂CHOHCH₂OH, -CH₂CH₂CHOH, -CH₂COHCH₃ und Hydroxybutylreste, wobei die Bindung von -X- zu R4 von der freien Valenz des betreffenden Kohlenstoffatom ausgeht
und R4 steht für:
1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann,
und A steht für ein physiologisch verträgliches organisches oder anorganisches Anion und wird an dieser Stelle stellvertretend für alle auch im Folgenden beschriebenen Strukturen definiert. Das Anion aller beschriebenen kationischen Verbindungen ist ausgewählt aus den Halogenidionen, Fluorid, Chlorid, Bromid, lodid, Sulfaten der allgemeinen Formel RSO₃-, worin R die Bedeutung von gesättigtem oder ungesättigtem Alkylresten mit 1 bis 4 Kohlenstoffatomen hat, oder anionischen Reste organischer Säuren wie Maleat, Fumarat, Oxalat, Tartrat, Citrat, Lactat oder Acetat.

Solche Produkte werden beispielsweise unter den Warenzeichen Rewoquat®, Stepantex®, Dehyquart®, Armocare® und Akypoquat® vertrieben. Die Produkte Armocare® VGH-70, Dehyquart® F-75, Dehyquart® C-4046, Dehyquart® L80, Dehyquart® F-30, Dehyquart® AU-35, Rewoquat® WE18, Rewoquat® WE38 DPG, Stepantex® VS 90 und Akypoquat® 131 sind Beispiele für diese Esterquats. Weitere erfindungsgemäß besonders bevorzugte Verbindungen der Formel (Tkat1-2) zählen zur Formel (Tkat1-2.1), den kationischen Betainestern. 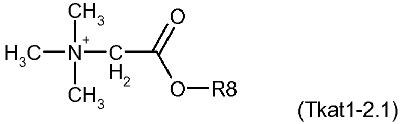 R8 entspricht in seiner Bedeutung R7.

Besonders bevorzugt sind die Esterquats mit den Handelsbezeichnungen Armocare® VGH-70, sowie Dehyquart® F-75, Dehyquart® L80, Stepantex® VS 90 und Akypoquat® 131.

Eine weitere Gruppe sind quartäre Imidazolinverbindungen. Die im Folgenden dargestellte Formel (Tkat2) zeigt die Struktur dieser Verbindungen.

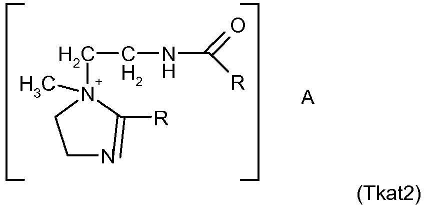

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat2) enthalten für R jeweils den gleichen Kohlenwasserstoffrest. Die Kettenlänge der Reste R beträgt bevorzugt 12 bis 21 Kohlenstoffatome. A steht für ein Anion wie zuvor beschrieben. Besonders erfindungsgemäße Beispiele sind beispielsweise unter den INCII - Bezeichnungen Quaternium-27, Quaternium-72, Quaternium-83 und Quaternium-91 erhältlich. Höchst bevorzugt ist erfindungsgemäß Quaternium-91.

Kationische Tenside der Formel (Tkat1-1) sind die dritte Gruppe bevorzugter quarternärer Ammoniumverbindungen.

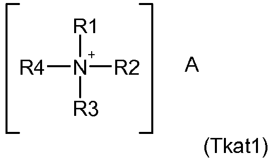

In der Formel (Tkat1) stehen R1, R2, R3 und R4 für jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1) sind Lauryltrimehtylammoniumchlorid, Cetyltrimethylammoniumchlorid, Cetyltrimethylammoniumbromid, Cetyltrimethylammoniummethosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat. Besonders bevorzugt sind Verbindungen mit mindestens einem Cetyl- oder Behenylrest im Molekül. Höchst bevorzugt sind Cetyltrimethylammonium und Behenyltrimethylammoniumsalze, allerhöchst bevorzugt Cetyltrimethylammoniumchlorid und Behenyltrimethylammoniumchlorid.

Äußerst bevorzugte Verfahren sind dadurch gekennzeichnet, daß die Umformungsmittel 0,15 bis 5 Gew.-%, vorzugsweise 0,11 bis 2,5 Gew.-%, weiter bevorzugt 0,12 bis 2 Gew.-%, noch weiter bevorzugt 0,13 bis 1 Gew.-% und insbesondere 0,15 bis 0,75 Gew.-% Alkyltrimethylammoniumchlorid(e), insbesondere Cetyltrimethylammoniumchlorid, enthalten. Alle zuvor genannten quarternären Ammoniumverbindungen sind kationischen Tenside und können einzeln oder in beliebigen Kombinationen miteinander verwendet werden, wobei Mengen zwischen 0,01 bis 10 Gew.%, bevorzugt Mengen von 0,01 bis 7,5 Gew.% und ganz besonders bevorzugt Mengen von 0,1 bis 5,0 Gew.% enthalten sind. Die allerbesten Ergebnisse werden dabei mit Mengen von 0,1 bis 3,0 Gew.% jeweils bezogen auf die Gesamtzusammensetzung des jeweiligen im erfindungsgemäßen Verfahren eingesetzten Umformungsmittels, erhalten. Diese Mengen werden auch nicht unter- oder überschritten, wenn Mischungen der kationischen Tenside verwendet werden.

Eine weitere bevorzugte Gruppe von Inhaltsstoffen der im erfindungsgemäßen Verfahren eingesetzten Umformungsmittel sind Vitamine, Provitamine oder Vitaminvorstufen.

Vitamine, Pro-Vitamine und Vitaminvorstufen sind dabei besonders bevorzugt, die den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.:
Vitamin B₁ (Thiamin)
Vitamin B₂ (Riboflavin)
Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Pantothensäure wird bevorzugt als Derivat in Form der stabileren Calciumsalze und Natriumsalze (Ca-Pantothenat, Na-Pantothenat) in der vorliegenden Erfindung eingesetzt.
Vitamin B₆ (Pyridoxin sowie Pyridoxamin und Pyridoxal).

Die genannten Verbindungen des Vitamin B -Typs insbesondere Vitamin B₃, B₅ und B₆, sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.

Vitamin C (Ascorbinsäure). Vitamin C wird in den im erfindungsgemäßen Verfahren eingesetzten Umformungsmitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den im erfindungsgemäßen Verfahren eingesetzten Umformungsmitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zusammensetzungen Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt.

Eine besonders bevorzugte Gruppe von Inhaltsstoffen in den im erfindungsgemäßen Verfahren eingesetzten Umformungsmittel sind die im folgenden genannten Betaine: Carnitin, Carnitintartrat, Carnitin Magnesiumcitrat, Acetylcarnitin, Betalaine, 1,1-Dimethyl-Prolin, Cholin, Cholinchlorid, Cholinbitartrat, Cholindihydrogencitrat und die in der Literatur als Betain bezeichnete Verbindung N,N,N-trimethylglycin.

Bevorzugt werden Carnitin, Histidin, Cholin sowie Betain verwendet. In einer besonders bevorzugten Ausführungsform der Erfindung wird als Wirkstoff L-Carnitintartrat eingesetzt.

In einer weiteren erfindungsgemäß bevorzugten Ausführungsform enthalten die im erfindungsgemäßen Verfahren eingesetzten Umformungsmittel Biochinone. In den im erfindungsgemäßen Verfahren eingesetzten Umformungsmitteln sind unter geeigneten Biochinonen ein oder mehrere Ubichinon(e) und/oder Plastochinon(e) zu verstehen. Die erfindungsgemäß bevorzugten Ubichinone weisen die folgende Formel auf:

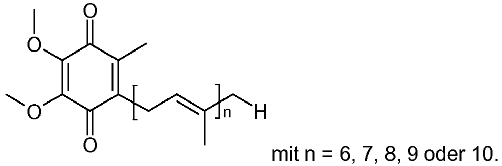

Das Coenzym Q-10 ist hierbei am bevorzugtesten.

Bevorzugte im erfindungsgemäßen Verfahren eingesetzten Umformungsmittel enthalten Purin und/oder Purinderivate in engeren Mengenbereichen. Hier sind im erfindungsgemäßen Verfahren eingesetzten Umformungsmittel vorzugsweise dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten. Erfindungsgemäß bevorzugte kosmetische Mittel sind dadurch gekennzeichnet, daß sie Purin, Adenin, Guanin, Harnsäure, Hypoxanthin, 6-Purinthiol, 6-Thioguanin, Xanthin, Coffein, Theobromin oder Theophyllin enthalten. In haarkosmetischen Zubereitungen ist Coffein am bevorzugtesten.

In einer weiteren bevorzugten Ausführungsform der vorliegenden Erfindung enthält das im erfindungsgemäßen Verfahren eingesetzten Umformungsmittel Ectoin ((*S*)-2-Methyl-1,4,5,6-tetrahydro-4-pyrimidincarbonsäure.

Erfindungsgemäß besonders bevorzugt sind Mittel, die - bezogen auf ihr Gewicht - 0,00001 bis 10,0 Gew.-%, vorzugsweise 0,0001 bis 5,0 Gew.-% und insbesondere 0,001 bis 3 Gew.-% der Wirkstoffe aus der Gruppe, die gebildet wird von Carnitin, Coenzym Q-10, Ectoin, ein Vitamin der B - Reihe, einem Purin und deren Derivaten oder physiologisch vertretbaren Salze enthalten.

Ein ganz besonders bevorzugter Pflegezusatz in den im erfindungsgemäßen Verfahren eingesetzten Umformungsmittel ist Taurin. Unter Taurin wird ausschließlich 2-Aminoethansulfonsäure und unter einem Derivat die explizit genannten Derivate des Taurines verstanden. Unter den Derivaten des Taurines werden N-Monomethyltaurin, N,N-Dimethyltaurin, Taurinlysylat, Taurintartrat, Taurinornithat, Lysyltaurin und Ornithyltaurin verstanden.

Besonders bevorzugt sind im erfindungsgemäßen Verfahren eingesetzten Umformungsmittel, die - bezogen auf ihr Gewicht - 0,0001 bis 10,0 Gew.-%, vorzugsweise 0,0005 bis 5,0 Gew.-%, besonders bevorzugt 0,001 bis 2,0 Gew.-% und insbesondere 0,001 bis 1,0 Gew.-% Taurin und/oder eines Derivates des Taurines enthalten.

Durch die Verwendung von Pflanzenextrakten als Pflegestoffe können die im erfindungsgemäßen Verfahren eingesetzten Umformungsmittel besonders naturnah und dennoch sehr effektiv in ihrer Pflegeleistung formuliert werden. Gegebenenfalls kann dabei sogar auf ansonsten übliche Konservierungsmittel verzichtet werden. Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa, Ingwerwurzel und ayurvedische Pflanzenextrakte wie beispielsweise Aegle Marmelos (Bilwa), Cyperus Rotundus (Nagar Motha), Emblica Officinalis (Amalki), Morida Citrifolia (Ashyuka), Tinospora Cordifolia (Guduchi), Santalum album, (Chandana), Crocus Sativus (Kumkuma), Cinnamonum Zeylanicum und Nelumbo Nucifera (Kamala), Süßgräser wie Weizen, Gerste, Roggen, Hafer, Dinkel, Mais, die verschiedenen Sorten der Hirse (Rispenhirse, Fingerhirse, Kolbenhirse als Beispiele), Zuckerrohr, Weidelgras, Wiesenfuchsschwanz, Glatthafer, Straußgras, Wiesenschwingel, Pfeifengras, Bambus, Baumwollgras, Lampenputzergräser, Andropogonodeae (Imperata Cylindrica auch Flammengras oder Cogon Gras genannt), Büffelgras, Schlickgräser, Hundszahngräser, Liebesgräser, Cymbopogon (Zitronengras), Oryzeae (Reis), Zizania (Wildreis), Strandhafer, Staudenhafer, Honiggräser, Zittergräser, Rispengräser, Quecken und Echinacea, insbesondere Echinacea purpurea (L.) Moench, aller Arten von Wein sowie Perikarp von Litchie chinensis bevorzugt.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Es hat sich gezeigt, daß bestimmte Proteolipide die Wirkung des erfindungsgemäßen Verfahrens noch weiter verstärken. Als weiteren Inhaltsstoff können die im erfindungsgemäßen Verfahren eingesetzten Umformungsmittel mindestens ein Proteolipid der Formel (P-I) enthalten

**R'-X-R"** (P-I),

in der
- R' für einen geradkettigen oder verzweigten, gesättigten oder ungesättigten Kohlenwasserstoffrest mit 11 bis 24 Kohlenstoffatomen steht,
- R" ein Protein, ein Peptid oder ein Proteinhydrolysat bedeutet,
- X für -C(O)O- oder -N⁺(R^{III}₂)R^{IV}- oder-N(R^{III})R^{IV}- oder -C(O)-N(R^{V})R^{VI}- steht,
- R^{III} -(CH₂)ₓ-CH₃ mit x = 0 - 22 bedeutet und
- R^{IV} -CH₂-CH(OH)-CH₂- oder -(CH₂)ₓ- mit x = 0 - 22 bedeutet;
- R^{V} und R^{V} unabhängig voneinander für -H oder -(CH₂)ₓ-CH₃ mit x = 0 - 22 stehen;
mit der Maßgabe, daß R" für Keratin oder ein Keratinhydrolysat steht, wenn X für -C(O)O- steht.

Vorzugsweise werden die Proteolipide innerhalb bestimmter Mengen in den im erfindungsgemäßen Verfahren eingesetzten Umformungsmitteln eingesetzt. Bevorzugte im erfindungsgemäßen Verfahren eingesetzten Umformungsmittel enthalten - bezogen auf ihr Gewicht - 0,01 bis 10 Gew.-%, vorzugsweise 0,02 bis 5 Gew.-%, besonders bevorzugt 0,05 bis 2,5 Gew.-%, weiter bevorzugt 0,1 bis 1 Gew.-% und insbesondere 0,15 bis 0,5 Gew.-% Proteolipid(e).

Der Rest R" in Formel (P-I) steht für ein Peptid oder ein Protein oder ein Proteinhydrolysat. Wenn X = -C(O)O-, wird R" aus der Gruppe Keratin oder Keratinhydrolysat gewählt.

Bevorzugte Reste R" sind Oligopetide, die mindestens eine Aminosäuresequenz Glu-Glu-Glu 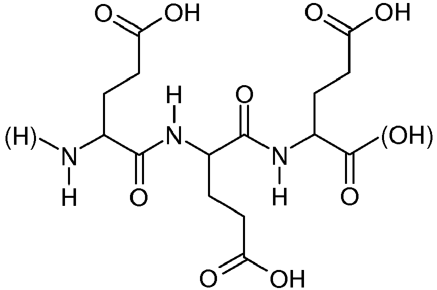 aufweisen, wobei die Amino-Gruppe frei oder protoniert und die Carboxy-Gruppen frei oder deprotoniert vorliegen können.

In dieser wie in allen nachstehenden Formeln bedeutet das eingeklammerte Wasserstoffatom der Aminogruppe ebenso wie die eingeklammerte Hydroxygruppe der Säurefunktion, daß die betreffenden Gruppen als solche vorhanden sein können (dann handelt es sich um ein Oligopeptid mit der betreffenden Anzahl an Aminosäuren wie dargestellt (in der vorstehenden Formel 3) oder aber, daß die Aminosäuresequenz in einem Oligopeptid vorliegt, das noch weitere Anminosäuren umfaßt - je nachdem, wo die weitere(n) Aminosäure(n) gebunden ist/sind, sind die eingeklammerten Bestandteile der o.g. Formel durch den/die weiteren Aminosäurerest(e) ersetzt.

Oligopeptide im Sinne der vorliegenden Anmeldung sind durch Peptid-Bindungen Säureamid-artig verknüpfte Kondensationsprodukte von Aminosäuren, die mindestens 3 und maximal 25 Aminosäuren umfassen

In bevorzugten im erfindungsgemäßen Verfahren eingesetzten Umformungsmitteln der vorstehend beschriebenen Ausfürhrungsform umfaßt das Oligopeptid (= der Rest R") 5 bis 15 Aminosäuren, vorzugsweise 6 bis 13 Aminosäuren, besonders bevorzugt 7 bis 12 Aminosäuren und insbesondere 8, 9 oder 10 Aminosäuren.

Je nachdem, ob weitere Aminosäuren an die Sequenz Glu-Glu-Glu gebunden sind und je nach Art dieser Aminosäuren sowie in Abhängigkeit von der Auswahl der Reste R' und ggf. R^{III} und R^{IV} kann die Molmasse des in den erfindungsgemäßen Mitteln enthaltenen Proteolipids variieren. Bevorzugt im erfindungsgemäßen Verfahren eingesetzte Umformungsmittel sind dadurch gekennzeichnet, daß das Proteolipid eine Molmasse von 1000 bis 30000 Da, vorzugsweise von 1250 bis 25000 Da, besonders bevorzugt von 1500 bis 20000 Da und insbesondere von 2000 bis 15000 Da aufweist.

Als Rest R" werden vorzugsweise Oligopeptide eingesetzt, die nicht allein aus den drei Glutaminsäuren bestehen, sondern weitere, an diese Sequenz gebundene Aminosäuren aufweisen. Diese weiteren Aminosäuren sind vorzugsweise aus bestimmten Aminosäuren ausgewählt, während bestimmte andere Vertreter erfindungsgemäß weniger bevorzugt sind.

So ist es bevorzugt, wenn der Rest R" der in den im erfindungsgemäßen Verfahren eingesetzten Umformungsmitteln eingesetzten Proteolipide kein Methionin enthält. Weiter bevorzugt ist es, wenn der Rest R" der in den erfindungsgemäßen Mitteln eingesetzten Proteolipide kein Cystein und/oder Cystin enthält.
Weiter bevorzugt ist es, wenn der Rest R" der in den im erfindungsgemäßen Verfahren eingesetzten Umformungsmitteln eingesetzten Proteolipide keine Asparaginsäure und/oder Asparagin enthält. Weiter bevorzugt ist es, wenn der Rest R" der in den erfindungsgemäßen Mitteln eingesetzten Proteolipide kein Serin und/oder Threonin enthält.

Demgegenüber ist es bevorzugt, wenn der Rest R" der in den im erfindungsgemäßen Verfahren eingesetzten Umformungsmitteln eingesetzten Proteolipide Tyrosin enthält. Weiter bevorzugt ist es, wenn der Rest R" der in den erfindungsgemäßen Mitteln eingesetzten Proteolipide Leucin enthält. Weiter bevorzugt ist es, wenn der Rest R" der in den erfindungsgemäßen Mitteln eingesetzten Proteolipide Isoleucin enthält. Weiter bevorzugt ist es, wenn der Rest R" der in den im erfindungsgemäßen Verfahren eingesetzten Umformungsmitteln eingesetzten Proteolipide Arginin enthält. Weiter bevorzugt ist es, wenn der Rest R" der in den im erfindungsgemäßen Verfahren eingesetzten Umformungsmitteln eingesetzten Proteolipide Valin enthält.

Als Rest R" besonders bevorzugte Oligopeptide bzw in den bevorzugten Oligopeptiden enthaltene Aminosäuresequenzen werden nachstehend beschrieben:
Ein besonders bevorzugtes Oligopeptid enthält zusätzlich Tyrosin, das vorzugsweise über seine Säurefunktion an die Glu-Glu-Glu-Sequenz gebunden ist. Bevorzugte im erfindungsgemäßen Verfahren eingesetzte Umformungsmittel sind daher dadurch gekennzeichnet, daß das in den Proteolipiden der Formel (I) als Rest R" enthaltene Oligopeptid mindestens eine Aminosäuresequenz Tyr-Glu-Glu-Glu 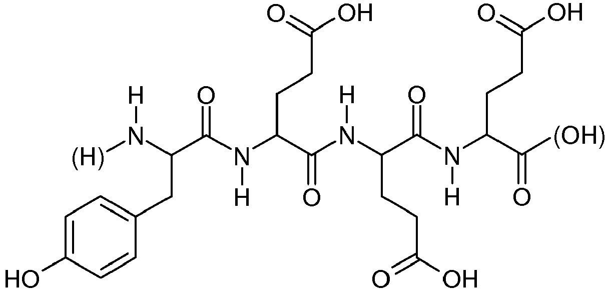 aufweist, wobei die Amino-Gruppe frei oder protoniert und die Carboxy-Gruppen frei oder deprotoniert vorliegen können.

Ein weiteres besonders bevorzugtes Oligopeptid enthält zusätzlich Isoleucin, das vorzugsweise über seine Aminofunktion an die Glu-Glu-Glu-Sequenz gebunden ist. Bevorzugte im erfindungsgemäßen Verfahren eingesetzte Umformungsmittel sind daher dadurch gekennzeichnet, daß das in den Proteolipiden der Formel (I) als Rest R" enthaltene Oligopeptid mindestens eine Aminosäuresequenz Glu-Glu-Glu-Ile 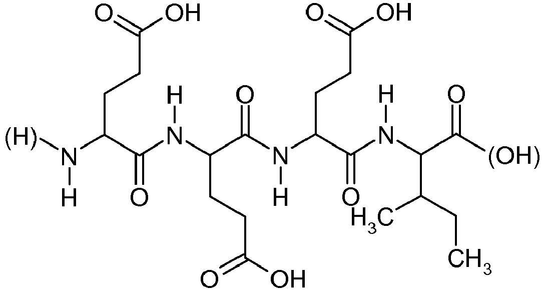 aufweist, wobei die Amino-Gruppe frei oder protoniert und die Carboxy-Gruppen frei oder deprotoniert vorliegen können.

Oligopeptide, die beide vorgenannten Aminosäuren (Tyrosin und Isoleucin) aufweisen, sind erfindungsgemäß bevorzugt. Bevorzugt sind im erfindungsgemäßen Verfahren eingesetzte Umformungsmittel, bei denen das in den Proteolipiden der Formel (I) als Rest R" enthaltene Oligopeptid mindestens eine Aminosäuresequenz Tyr-Glu-Glu-Glu-Ile 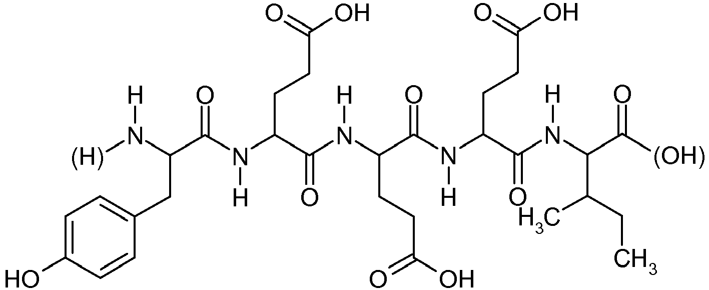 aufweist, wobei die Amino-Gruppe frei oder protoniert und die Carboxy-Gruppen frei oder deprotoniert vorliegen können.

Weiter bevorzugte Oligopeptide enthalten zusätzlich Arginin, das vorzugsweise an Isoleucin gebunden vorliegt. Bevorzugte im erfindungsgemäßen Verfahren eingesetzte Umformungsmittel sind daher dadurch gekennzeichnet, daß das in den Proteolipiden der Formel (I) als Rest R" enthaltene Oligopeptid mindestens eine Aminosäuresequenz Tyr-Glu-Glu-Glu-Ile-Arg 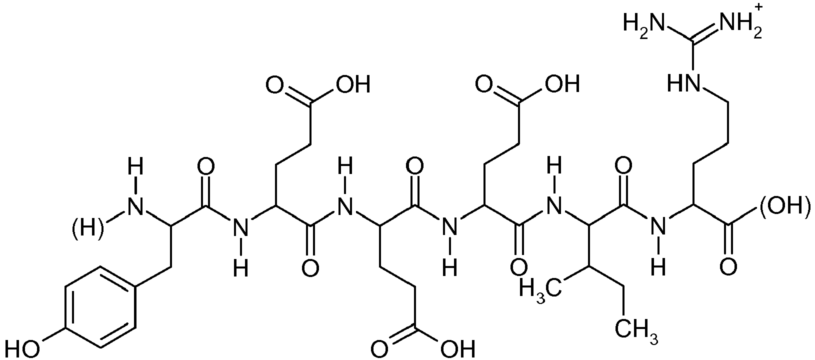 aufweist, wobei die Amino-Gruppen frei oder protoniert und die Carboxy-Gruppen frei oder deprotoniert vorliegen können.

Noch weiter bevorzugte Oligopeptide enthalten zusätzlich Valin, das vorzugsweise an das Arginin gebunden vorliegt. Bevorzugte im erfindungsgemäßen Verfahren eingesetzte Umformungsmittel sind daher dadurch gekennzeichnet, daß das in den Proteolipiden der Formel (I) als Rest R" enthaltene Oligopeptid mindestens eine Aminosäuresequenz Tyr-Glu-Glu-Glu-Ile-Arg-Val 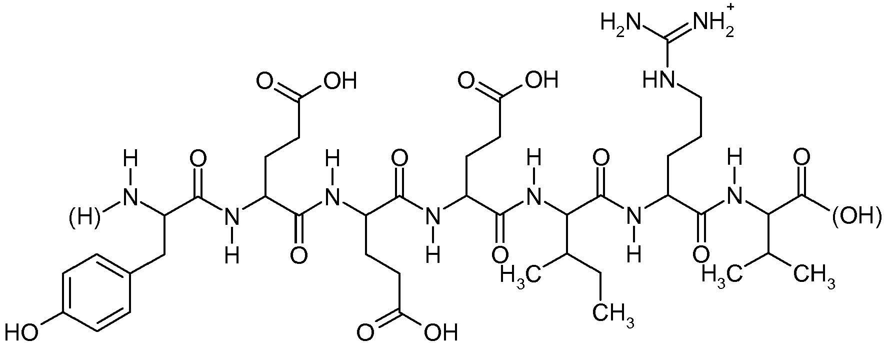 aufweist, wobei die Amino-Gruppen frei oder protoniert und die Carboxy-Gruppen frei oder deprotoniert vorliegen können.

Noch weiter bevorzugte Oligopeptide enthalten zusätzlich Leucin, das vorzugsweise an Valin gebunden vorliegt. Bevorzugte im erfindungsgemäßen Verfahren eingesetzte Umformungsmittel sind dadurch gekennzeichnet, daß das in den Proteolipiden der Formel (I) als Rest R" enthaltene Oligopeptid mindestens eine Aminosäuresequenz Tyr-Glu-Glu-Glu-Ile-Arg-Val-Leu 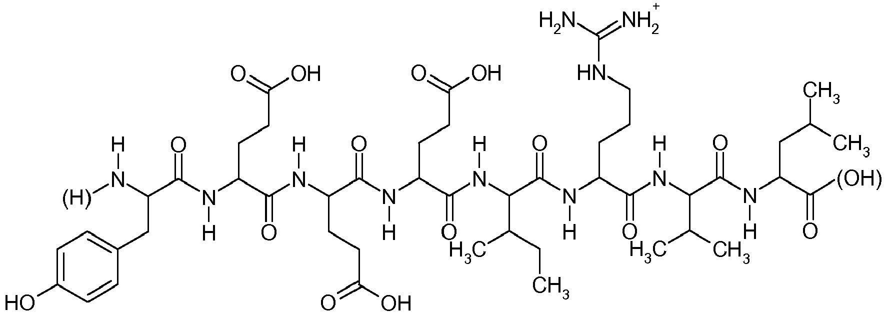 aufweist, wobei die Amino-Gruppen frei oder protoniert und die Carboxy-Gruppen frei oder deprotoniert vorliegen können.

Insbesondere bevorzugte Oligopeptide enthalten zusätzlich Leucin, das vorzugsweise an das Tyrosin gebunden vorliegt. Bevorzugte im erfindungsgemäßen Verfahren eingesetzte Umformungsmittel sind dadurch gekennzeichnet, daß das in den Proteolipiden der Formel (I) als Rest R" enthaltene Oligopeptid mindestens eine Aminosäuresequenz Leu-Tyr-Glu-Glu-Glu-Ile-Arg-Val-Leu 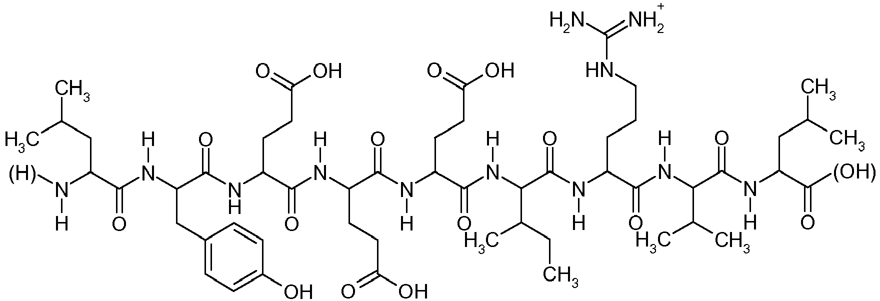 aufweist, wobei die Amino-Gruppen frei oder protoniert und die Carboxy-Gruppen frei oder deprotoniert vorliegen können.

Zusammenfassend sind insbesondere im erfindungsgemäßen Verfahren eingesetzte Umformungsmittel bevorzugt, die mindestens ein Proteolipd der Formel (I) enthalten, in der R" mindestens eine Aminosäuresequenz Leu-Tyr-Glu-Glu-Glu-Ile-Arg-Val-Leu 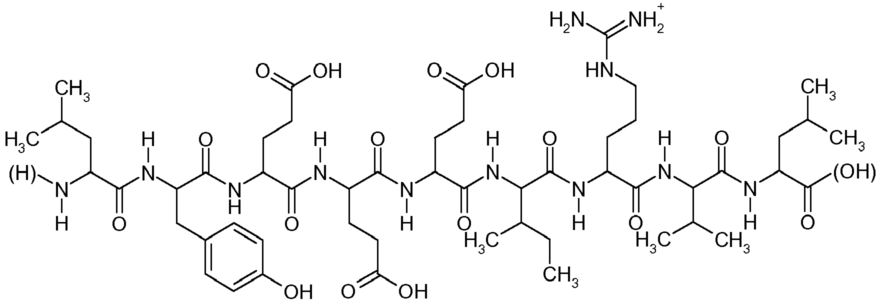 aufweist, wobei die Amino-Gruppen frei oder protoniert und die Carboxy-Gruppen frei oder deprotoniert vorliegen können.

Wie bereits erwähnt, wird R" aus der Gruppe Keratin oder Keratinhydrolysat gewählt, wenn X = -C(O)O- gilt.

In allen anderen Fällen kann der Rest R" in Formel (P-I) für ein Peptid oder ein Protein oder ein Proteinhydrolysat stehen, wobei Proteinhydrolysate bevorzugt sind. Proteinhydrolysate sind Produktgemische, die durch sauer, basisch oder enzymatisch katalysierten Abbau von Proteinen (Eiweißen) erhalten werden. Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Vorzugsweise wird unabhängig von der Wahl des X in Formel (P-I) der Rest R" aus Keratin oder keratinhydrolxysaten ausgewählt. Bevorzugte erfindungsgemäße Kosmetische Mittel sind dadurch gekennzeichnet, daß sie mindestens ein Proteolipid der Formel (P-I) enthalten, in der R" für Keratin oder ein Keratinhydrolysat steht.

Insbesondere sind im erfindungsgemäßen Verfahren eingesetzte Umformungsmittel bevorzugt, die mindestens ein Proteolipd der Formel (P-I) enthalten, in der R^{III}-CH₃ bedeutet und R^{IV} für-(CH₂)ₓ- mit x = 0, 1, 2, 3, 4, 5, 6, 7, 8 steht.

Weiter sind besonders bevorzugte im erfindungsgemäßen Verfahren eingesetzte Umformungsmittel dadurch gekennzeichnet, daß sie mindestens ein Proteolipd der Formel (I) enthalten, in der X für - N⁺(CH₃)₂-CH₂-CH(OH)-CH₂- und R' für -(CH₂)₁₇-CH₃ steht.

Ebenfalls weiter bevorzugte im erfindungsgemäßen Verfahren eingesetzte Umformungsmittel dadurch gekennzeichnet, daß sie mindestens ein Proteolipd der Formel (P-I) enthalten, in der X für -C(O)-O- und R' für -(CH₂)₁₇-CH₃ steht.

Es hat sich als vorteilhaft erwiesen, zusätzlich zu den Proteolipiden Proteinhydrolysate einzusetzen. Diese verstärken die Wirkung der Proteolipide und werden ihrerseits in ihren Effekten verstärkt. Die Proteinhydrolysate wurden weiter oben als Rest R" detailliert beschrieben. Zusammenfassend sind im erfindungsgemäßen Verfahren eingesetzte Umformungsmittel bevorzugt, die zusätzlich - bezogen auf ihr Gewicht - 0,01 bis 10 Gew.-%, vorzugsweise 0,05 bis 7 Gew.-%, besonders bevorzugt 0,1 bis 5 Gew.-%, weiter bevorzugt 0,25 bis 2,5 Gew.-% und insbesondere 0,5 bis 2,0 Gew.-% Proteinhydrolysat(e), vorzugsweise Keratinhydrolysat(e) enthalten.

In Schritt (ii) des erfindungsgemäßen Verfahrens werden die Fasern nicht gespült, d.h. das Umformungsmittel verbleibt bei der Umformung auf den Fasern. Die Fasern können feucht umgeformt werden, es ist aber auch möglich, die Fasern nach der Applikation des Umformungsmittel und bevor dem Umformungsvorgang zu trocknen.

Bevorzugte erfindungsgemäße Verfahren sind dadurch gekennzeichnet, daß die Fasern in Schritt (ii) getrocknet werden.

Die Trocknung kann mittels eines Handtuches durch Frottieren oder vorzugsweise durch Anwendung von Heißluft, insbesondere durch Fönen, erfolgen.

Anschließend an die Behandlung mit dem Umformungsmittel werden die Fasern verformt {Schritt (iii) des erfindungsgemäßen Verfahrens} werden. Die Verformung in Schritt (iii) kann durch Wärme unterstützt werden, beispielsweise durch beheizte Lockenwickler oder - besonders bevorzugt - durch Anwendung eine Glätteisens.

Erfindungsgemäße Verfahren, bei denen die Fasern in Schritt (iii) einer Wärmebehandlung bei einer Temperatur von 50°C bis 350°C (vorzugsweise 80°C bis 280°C, besonders bevorzugt 100°C bis 250°C, weiter bevorzugt 140°C bis 220°C) unterworfen werden, sind erfindungsgemäß bevorzugt.

## Patentansprüche

1. Verfahren zur Umformung, insbesondere zur Glättung, keratinhaltiger Fasern, insbesondere menschlicher Haare, in welchem
(i) ein Umformungsmittel, enthaltend Carbocystein und/oder seine Salze auf die keratinischen Fasern aufgetragen und dort belassen wird
(ii) die Fasern nach einer Einwirkzeit
- nicht gespült werden,
- gegebenenfalls getrocknet werden,
(iii) die Fasern unter der Einwirkung von Wärme mechanisch verformt werden, wobei die Fasern einer Wärmebehandlung bei einer Temperatur von 80°C bis 280°C unterworfen werden,
**dadurch gekennzeichnet, dass** im Schritt (i) ein Umformungsmittel aufgetragen wird, welches bezogen auf sein Gewicht
- 0,1 bis 7,5 Gew.-% Carbocystein enthält
- 40 bis 92,5 Gew.-% Wasser enthält
- 0,15 bis 5 Gew.-% quartäre Ammoniumverbindung(en) aus der Gruppe
i) der Tetraalkylammoniumhalogenide und/oder
ii) der Esterquat und/oder
iii) der quarternären Imidazoline der Formel (Tkat2) 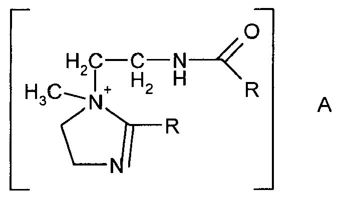 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
v) Poly(methacryloyloxyethyltrimethylammoniumverbindungen) und/oder;
vi) quaternisierten Cellulose-Derivaten, insbesondere Polyquaternium 10, Polyquaternium-24 Polyquaternium-27, Polyquaternium-67, Polyquaternium-72, und/oder
vii) kationische Alkylpolyglycoside und/oder
viii) kationisiertem Honig und/oder
ix) kationischen Guar-Derivaten und/oder
x) polymeren Dimethyldiallylammoniumsalzen und deren copolymeren mit Estern und Amiden von Acrylsäure und Methacrylsäure, insbesondere Polyquaternium-7 und/oder
xi) copolymeren des Vinylpyrrolidons mit quaternierten Derivaten des Dialkylaminoalkylacrylats und -methacrylats, insbesondere Polyquaternium-11 und/oder
xii) Vinylpyrrolidon-Vinylimidazoliummethochlorid-Copolymeren, insbesondere Polyquaternium-16 und/oder
xiii) quaterniertem Polyvinylalkohol und/oder
xiv) Polyquaternium-74,
xv) kationischen Alkyloligoglucosiden und/oder
xvi) Polyquaternium-71
sowie deren Mischungen enthält.

2. Verfahren nach Anspruch 1, **dadurch gekennzeichnet, daß** im Schritt (i) ein Umformungsmittel aufgetragen wird, welches bezogen auf sein Gewicht 0,25 bis 5 Gew.-%, noch weiter bevorzugt 0,5 bis 2,5 Gew.-% und insbesondere 0,55 bis 2 Gew.-% Carbocystein enthält.

3. Verfahren nach einem der Ansprüche 1 oder 2, **dadurch gekennzeichnet, daß** im Schritt (i) ein Umformungsmittel aufgetragen wird, welches bezogen auf sein Gewicht 50 bis 90 Gew.-%, noch weiter bevorzugt 60 bis 87,5 Gew.-% und insbesondere 70 bis 85 Gew.-% Wasser enthält.

4. Verfahren nach einem der Ansprüche 1 bis 3, **dadurch gekennzeichnet, daß** im Schritt (i) ein Umformungsmittel aufgetragen wird, welches bezogen auf sein Gewicht 0,2 bis 4 Gew.-%, noch weiter bevorzugt 0,25 bis 3 Gew.-% und insbesondere 0,3 bis 1 Gew.-% quartäre Ammoniumverbindung(en) aus der Gruppe
i) der Tetraalkylammoniumhalogenide und/oder
ii) der Esterquats und/oder
iii) der quarternären Imidazoline der Formel (Tkat2) 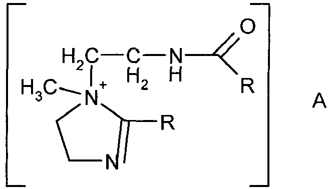 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
v) Poly(methacryloyloxyethyltrimethylammoniumverbindungen) und/oder;
vi) quaternisierten Cellulose-Derivaten, insbesondere Polyquaternium 10, Polyquaternium-24, Polyquaternium-27, Polyquaternium-67, Polyquaternium-72, und/oder
vii) kationische Alkylpolyglycoside und/oder
viii) kationisiertem Honig und/oder
ix) kationischen Guar-Derivaten und/oder
x) polymeren Dimethyldiallylammoniumsalzen und deren Copolymeren mit Estern und Amiden von Acrylsäure und Methacrylsäure, insbesondere Polyquaternium-7 und/oder
xi) Copolymeren des Vinylpyrrolidons mit quaternierten Derivaten des Dialkylaminoalkylacrylats und -methacrylats, insbesondere Polyquaternium-11 und/oder
xii) Vinylpyrrolidon-Vinylimidazoliummethochlorid-Copolymeren, insbesondere Polyquaternium-16 und/oder
xiii) quaterniertem Polyvinylalkohol und/oder
xiv) Polyquaternium-74,
xv) kationischen Alkyloligoglucosiden und/oder
xvi) Polyquaternium-71
sowie deren Mischungen enthält.

5. Verfahren nach einem der Ansprüche 1 bis 4, **dadurch gekennzeichnet, daß** die Fasern in Schritt (ii) getrocknet werden.

6. Verfahren nach einem der Ansprüche 1 bis 5, **dadurch gekennzeichnet, daß** die Fasern in Schritt (iii) einer Wärmebehandlung bei einer Temperatur von 100°C bis 250°C, weiter bevorzugt 140°C bis 220°C unterworfen werden.

## Claims

1. A method for styling, in particular for straightening, keratin fibers, in particular human hair, in which
(i) a styling agent comprising carbocysteine and/or its salts is applied to the keratin fibers and left thereon,
(ii) the fibers, after a contact time,
- are not rinsed,
- are optionally dried,
(iii) the fibers are mechanically deformed under the influence of heat, the fibers being subjected to heat treatment at a temperature of from 80°C to 280°C,
**characterized in that**, in step (i), a styling agent is applied which contains, based on its weight,
- from 0.1 to 7.5 wt.% carbocysteine,
- from 40 to 92.5 wt.% water,
- from 0.15 to 5 wt.% quaternary ammonium compound(s) selected from the group of
i) tetraalkylammonium halides and/or
ii) esterquats and/or
iii) quaternary imidazolines of formula (Tkat2) 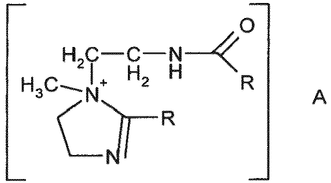 in which the functional groups R, independently of one another, each represent a saturated or unsaturated, linear or branched hydrocarbon functional group having a chain length of 8 to 30 carbon atoms and A represents a physiologically acceptable anion, and/or
iv) poly(methacryloyloxyethyltrimethylammonium) compounds and/or
v) quaternized cellulose derivatives, in particular Polyquaternium-10, Polyquaternium-24, Polyquaternium-27, Polyquaternium-67, Polyquaternium-72 and/or
vi) cationic alkyl polyglycosides and/or
vii) cationized honey and/or
viii) cationic guar derivatives and/or
ix) polymeric dimethyldiallylammonium salts and copolymers thereof with esters and amides of acrylic acid and methacrylic acid, in particular Polyquaternium-7 and/or
x) copolymers of vinylpyrrolidone with quaternized derivatives of dialkylaminoalkyl acrylate and methacrylate, in particular Polyquaternium-11 and/or
xi) vinylpyrrolidone-vinylimidazolium methochloride copolymers, in particular Polyquaternium-16 and/or
xii) quaternized polyvinyl alcohol and/or
xiii) Polyquaternium-74,
xiv) cationic alkyl oligoglucosides and/or
xv) Polyquaternium-71
and mixtures thereof.

2. The method according to claim 1, **characterized in that**, in step (i), a styling agent is applied which contains, based on its weight, from 0.25 to 5 wt.%, even more preferably from 0.5 to 2.5 wt.% and in particular from 0.55 to 2 wt.% carbocysteine.

3. The method according to one of claims 1 or 2, **characterized in that**, in step (i), a styling agent is applied which contains, based on its weight, from 50 to 90 wt.%, even more preferably from 60 to 87.5 wt.% and in particular from 70 to 85 wt.% water.

4. The method according to one of claims 1 to 3, **characterized in that**, in step (i), a styling agent is applied which contains, based on its weight, from 0.2 to 4 wt.%, even more preferably from 0.25 to 3 wt.% and in particular from 0.3 to 1 wt.% quaternary ammonium compound(s) selected from the group of
i) tetraalkylammonium halides and/or
ii) esterquats and/or
iii) quaternary imidazolines of formula (Tkat2) 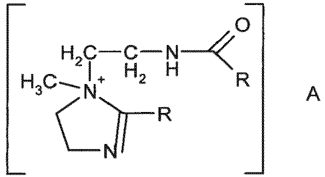 in which the functional groups R, independently of one another, each represent a saturated or unsaturated, linear or branched hydrocarbon functional group having a chain length of 8 to 30 carbon atoms, and A represents a physiologically acceptable anion, and/or
iv) poly(methacryloyloxyethyltrimethylammonium) compounds and/or
v) quaternized cellulose derivatives, in particular Polyquaternium-10, Polyquaternium-24, Polyquaternium-27, Polyquaternium-67, Polyquaternium-72 and/or
vi) cationic alkyl polyglycosides and/or
vii) cationized honey and/or
viii) cationic guar derivatives and/or
ix) polymeric dimethyldiallylammonium salts and copolymers thereof with esters and amides of acrylic acid and methacrylic acid, in particular Polyquaternium-7 and/or
x) copolymers of vinylpyrrolidone with quaternized derivatives of dialkylaminoalkyl acrylate and methacrylate, in particular Polyquaternium-11 and/or
xi) vinylpyrrolidone-vinylimidazolium methochloride copolymers, in particular Polyquaternium-16 and/or
xii) quaternized polyvinyl alcohol and/or
xiii) Polyquaternium-74,
xiv) cationic alkyl oligoglucosides and/or
xv) Polyquaternium-71
and mixtures thereof.

5. The method according to one of claims 1 to 4, **characterized in that** the fibers are dried in step (ii).

6. The method according to one of claims 1 to 5, **characterized in that** the fibers are subjected to a heat treatment in step (iii) at a temperature of from 100°C to 250 °C, more preferably from 140 °C to 220 °C.

## Revendications

1. Procédé de mise en forme, en particulier de lissage, de fibres kératiniques, en particulier de cheveux humains, dans lequel
(i) un agent de mise en forme contenant de la carbocystéine et/ou des sels de celle-ci est appliqué et laissé sur les fibres kératiniques
(ii) les fibres, après un temps de pose,
- ne sont pas rincées,
- sont éventuellement séchées,
(iii) les fibres sont mises en formes mécaniquement sous l'action de la chaleur, les fibres étant soumises à un traitement thermique à une température allant de 80 °C à 280 °C,
**caractérisé en ce qu'**à l'étape (i), on applique un agent de mise en forme qui, rapporté à son poids,
- contient de 0,1 à 7,5 % en poids de carbocystéine,
- contient de 40 à 92,5 % en poids d'eau,
- contient de 0,15 à 5 % en poids de composé(s) d'ammonium quaternaire du groupe
i) des halogénures de tétraalkylammonium et/ou
ii) des esterquats et/ou
iii) des imidazolines quaternaires de Formule (Tkat2) 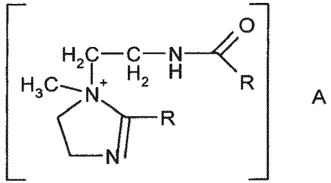 dans laquelle les résidus R représentent, indépendamment l'un de l'autre, respectivement un résidu d'hydrocarbure saturé ou non-saturé, linéaire ou ramifié, avec une longueur de chaîne de 8 à 30 atomes de carbone et A représente un anion physiologiquement compatible, et/ou contient
iv) des composés de poly (méthacryloyloxyéthyltriméthylammonium) et/ou ;
v) des dérivés de cellulose quaternisés, en particulier le Polyquaternium-10, le Polyquaternium-24, le Polyquaternium-27, le Polyquaternium-67, le Polyquaternium-72, et/ou
vi) des alkylpolyglycosides cationiques et/ou
vii) du miel cationisé et/ou
viii) des dérivés de guar cationiques et/ou
ix) des sels de diméthyldiallylammonium polymères et leurs copolymères avec des esters et des amides d'acide acrylique et d'acide méthacrylique, en particulier le Polyquaternium-7 et/ou
x) des copolymères de vinylpyrrolidone avec des dérivés quaternisés d'acrylate de dialkylaminoalkyle et de méthacrylate de dialkylaminoalkyle, en particulier le Polyquaternium-11 et/ou
xi) des copolymères de vinylpyrrolidone - méthochlorure de vinylimidazolium, en particulier
le Polyquaternium-16 et/ou
xii) un alcool polyvinylique quaternisé et/ou
xiii) le Polyquaternium-74,
xiv) des alkyloligoglucosides cationiques et/ou
xv) le Polyquaternium-71
ainsi que des mélanges de ceux-ci.

2. Procédé selon la revendication 1, **caractérisé en ce qu'**à l'étape (i), on applique un agent de mise en forme qui, rapporté à son poids, contient de 0,25 à 5 % en poids, de façon encore plus préférée de 0,5 à 2,5 % en poids et en particulier de 0,55 à 2 % en poids de carbocystéine.

3. Procédé selon l'une quelconque des revendications 1 ou 2, **caractérisé en ce qu'**à l'étape (i), on applique un agent de mise en forme qui, rapporté à son poids, contient de 50 à 90 % en poids, de façon encore plus préférée de 60 à 87,5 % en poids et en particulier de 70 à 85 % en poids d'eau.

4. Procédé selon l'une quelconque des revendications 1 à 3, **caractérisé en ce qu'**à l'étape (i), on applique un agent de mise en forme qui, rapporté à son poids, contient de 0,2 à 4 % en poids, de façon encore plus préférée de 0,25 à 3 % en poids et en particulier de 0,3 à 1 % en poids de
composé(s) d'ammonium quaternaire du groupe
i) des halogénures de tétraalkylammonium et/ou
ii) des esterquats et/ou
iii) des imidazolines quaternaires de Formule (Tkat2) 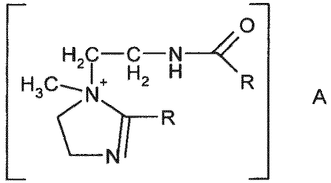 dans laquelle les résidus R représentent, indépendamment l'un de l'autre, respectivement un résidu d'hydrocarbure saturé ou non-saturé, linéaire ou ramifié, avec une longueur de chaîne de 8 à 30 atomes de carbone et A représente un anion physiologiquement compatible, et/ou contient
iv) des composés de poly (méthacryloyloxyéthyltriméthylammonium) et/ou ;
v) des dérivés de cellulose quaternisés, en particulier le Polyquaternium-10, le Polyquaternium-24, le Polyquaternium-27, le Polyquaternium-67, le Polyquaternium-72, et/ou
vi) des alkylpolyglycosides cationiques et/ou
vii) du miel cationisé et/ou
viii) des dérivés de guar cationiques et/ou
ix) des sels de diméthyldiallylammonium polymères et leurs copolymères avec des esters et des amides d'acide acrylique et d'acide méthacrylique, en particulier le Polyquaternium-7 et/ou
x) des copolymères de vinylpyrrolidone avec des dérivés quaternisés d'acrylate de dialkylaminoalkyle et de méthacrylate de dialkylaminoalkyle, en particulier le Polyquaternium-11 et/ou
xi) des copolymères de vinylpyrrolidone - méthochlorure de vinylimidazolium, en particulier le Polyquaternium-16 et/ou
xii) un alcool polyvinylique quaternisé et/ou
xiii) le Polyquaternium-74,
xiv) des alkyloligoglucosides cationiques et/ou
xv) le Polyquaternium-71
ainsi que des mélanges de ceux-ci.

5. Procédé selon l'une quelconque des revendications 1 à 4, **caractérisé en ce qu'**à l'étape (ii), les fibres sont séchées.

6. Procédé selon l'une quelconque des revendications 1 à 5, **caractérisé en ce qu'**à l'étape (iii), les fibres sont soumises à un traitement thermique à une température allant de 100°C à 250°C, plus préférablement de 140°C à 220°C.
